Europäisches Patentamt

European Patent Office

Office européen des brevets

(11) Publication number: **0 235 886**
**A2**

# (12) EUROPEAN PATENT APPLICATION

(21) Application number: **87300343.8**

(22) Date of filing: **16.01.87**

(51) Int. Cl.4: **B01J 27/047** , **C07C 29/15**

(30) Priority: **04.02.86 GB 8602654**

(43) Date of publication of application:
**09.09.87 Bulletin 87/37**

(84) Designated Contracting States:
**DE FR NL**

(71) Applicant: **Coal Industry (Patents) Limited**
**Hobart House Grosvenor Place**
**London SW1X 7AE(GB)**

(72) Inventor: **Gavin, Derek Gabriel**
**11 The Napping**
**Longhope Gloucestershire(GB)**
Inventor: **Richards, David Gareth**
**53 Roman Road**
**Cheltenham Gloucestershire(GB)**

(74) Representative: **Wood, John Irwin**
**NATIONAL COAL BOARD Hobart House**
**Grosvenor Place**
**London SW1X 7AE(GB)**

(54) **Catalyst and its use.**

(57) A catalyst particularly effective for producing mixed alcohols in high yields and with good selectivity from synthesis gas, eg for gasoline blending, is produced by contacting ammonium thiomolybdate with an active carbon support so as to deposit $MoS_3$, depositing a potassium compound, and activating by heating the catalyst in a reducing atmosphere.

EP 0 235 886 A2

## CATALYST AND ITS USE

This invention concerns a catalyst and its use, and more especially concerns a catalyst for the production of alcohols from synthesis gas.

It is being increasingly recognised that, with the removal of lead compounds from motor gasoline, a favoured alternative to maintain high antiknock properties is the use of mixed alcohols. The actual desirable mix of alcohols will be discussed in more detail below.

It is known to make mixed alcohols from synthesis gas, by passing the gas over a catalyst. A variety of catalysts have been disclosed, mainly based on iron oxide, copper with cobalt, rhodium or molybdenum. For example, the Synol process employed an iron oxide/potassium oxide catalyst to produce a mixture of alcohols (55%) and hydrocarbons (40%). The mixture boiled in the range 34° to 360°C, making them unsuitable for blending into modern gasoline. More recent processes published in the patent literature have concentrated on promoted rhodium catalysts, mainly on silica supports. Rhodium is, however, extremely expensive and is not particularly selective for producing alcohols at high rates because low value hydrocarbons are simultaneously produced.

It is also known to use molybdenum sulphide as a catalyst for making liquid hydrocarbons and organic oxygenates. For example, USP 2,490,488 discloses $MoS_2$ modified by the addition of alkali metal compounds. European Patent Application No 0 119609 (Dow) discloses a number of catalysts, both supported and bulk catalysts, and Examples 9 and 18 relate to $MoS_2$ promoted with potassium carbonate. Example 9 discloses 32% $MoS_2$ and 7.5% $K_2CO_3$ on active carbon and dried at 300°C in nitrogen yielding 121g/h/kg catalyst of alcohols at a selectivity of 91.6% ($CO_2$-free basis). Example 18 discloses a clay-supported catalyst containing 66% $MoS_2$ and 10% $K_2CO_3$ which yields 367g/h/kg catalyst of alcohol at 87% selectivity.

Another recent European Application, No 0 149 255 (Union Carbide), also disclosed the production of alcohols using alkali-promoted $MoS_2$. The best results were disclosed in Examples 8 and 21, both giving a yield of 256g/h/kg at 80% selectivity using unsupported $MoS_2$ with cesium promoters. A mixture of unsupported $MoS_2$ and KOH gave 221g/h/kg at 80% selectivity.

USP 4,243,553 discloses the decomposition of ammonium thiomolybdate to yield high surface area $MoS_2$ for use in water gas shift and methanation reactions. No mention is made of alcohols in this document, but it is noted that reaction temperatures are relatively high.

We have assessed the state of the art as still showing a need for an improved catalyst which would be used in a commercially acceptable process for economically making mixed alcohols in the C1 -C4 or C1 - C6 range with a composition suitable for blending into gasoline. It is estimated that commercially acceptable production rates could be 700g/hr/kg catalyst, or more, at selectivities of at least 85% and minimal water and hydrocarbon production. The catalyst should be resistant to sulphur poisoning.

The composition of alcohols is important if the use is in gasoline blending. Methanol alone exhibits problems associated with volatility of the blended gasoline, with a significant increase in the quantity of the gasoline boiling below 70°C, with a serious reduction in Motor Octane Number and also a serious reduction in water tolerance before phase separation is experienced, noticably at low temperatures. While blending with mixed alcohols alleviates these problems, it is desirable to minimise methanol content in any mixed alcohol product, and hence we consider that production of the higher alcohols such as propanol and butanol is important.

The present invention provides a promoted $MoS_2$ catalyst which overcomes the prior art low yields and selectivities. The invention provides a catalyst produced by contacting an active carbon support with an aqueous solution of sufficient concentration of ammonium thiomolybdate to give a loading of 10 to 25% by wt of $MoS_2$ in the final catalyst, by analysis, at a temperature of 0-50°C, depositing sufficient of potassium carbonate or hydroxide or a potassium salt other than a salt containing chlorine in its anion to give a loading of 5 -12% by wt, calculated as $K_2CO_3$, and activating the catalyst by heating in a reducing atmosphere at a temperature of 400 to 500°C. The invention also provides a process for the production of the catalyst, as described above.

The invention further provides a process for the production of mixed alcohols comprising the passage of a mixture of hydrogen and carbon monoxide in a volume ratio of from 0.2 to 2:1 over a catalyst according to the invention, at a temperature of 260 to 325°C and a pressure of at least 50 bar, and at a Gas Hourly Space Velocity of from 1000 to 50,000 h-1.

The catalyst will conveniently be described hereinafter as containing $MoS_2$ and $K_2CO_3$ although it is not known whether there is any combination between the molybdenum sulphide and the potassium salt during the activation steps. Preferred potassium salts include those of the organic acids, sulphate, sulphide, nitrate and hydrogen carbonate and the carbonate and hydroxide are especially preferred. The potassium salt may be deposited from aqueous solution on to the active carbon support before, after or simultaneously with the deposition of the molybdenum sulphide. The preferred loading of potassium, calculated as $K_2CO_3$ is from 6 to 10% by wt.

Suitable active carbon supports are commercially available and should have a surface area of at least 200m2/g, preferably at least 500m2/g.

The preparation of ammonium thiomolybdates is known. It is believed that contact with active carbon causes the deposition of molybdenum trisulphide on to the carbon, and this is reduced during activation to $MoS_2$. Providing that the final loading of $MoS_2$ is not greater than about 25% by weight, the contacting process will provide complete decomposition of the thiomolybdate to ammonia and $MoS_3$, which is deposited on the carbon. This has the particularly beneficial result that disposal problems associated with dilute heavy metal solutions are largely eliminated. It is important to operate within the stated temperature range, preferably about room temperature, since higher temperatures can cause sintering of $MoS_3$ and loss of eventual activity. It is to be noted that the prior art concerned with the production of $MoS_2$ catalysts for synthesis gas conversion generally recommend the so-called "incipient wetness" technique for depositing the molybdenum disulphide onto a carrier. The present invention does not utilise this method for the absorption of the $MoS_3$. Preferred loadings for $MoS_2$ are in the range 15 to 20% by wt.

An essential part of the preparation of the catalyst is the activation step. The catalyst is pyrophoric and will combust if heated in air. Additionally, heating under reducing conditions outside the stated temperature range has disadvantageous effects on the catalyst activity. Preferred temperatures are in the range 425 - 450°C, and heating is suitably carried out for several hours. Synthesis gas can conveniently be used for activation.

The use of the catalyst to convert synthesis gas to alcohol is preferably carried out at a temperature of 275 to 300°C and at a pressure of 100 to 150 bar. Preferred GHSV's are from 3.000 -50,000 h-1, especially 5,000 -20,000 h-1. In general, the alcohols yield improves with increasing GHSV while gas make is slightly reduced. The catalyst may exhibit a falling off of activity during an initial period, this can be compensated for by raising the temperature slightly, eg. in 5°C steps, until a steady activity is obtained.

We have discussed that the catalysts of the invention generate C2 + alcohols preferentially at low hydrogen to carbon ratios, thus increasing the value of the product substantially for gasoline blending. Preferred $h_2$:CO ratios are in the range 0.5 to 1:1. The major economic source of synthesis gas at the moment is from natural gas, but it is impossible to obtain $H_2$:CO ratios below 1:1 from natural gas, even utilising a shift reaction, except possible by expensive cryogenic methods. Coals of various types, however, can be gasified, eg in the Kopper -Totzek process, to yield initial $H_2$:CO ratios of approximately 0.7:1. It may also be possible to gasify heavy petroleum residues, tars and heavy oils to yield suitable starting materials. It should be noted that certain gasifying processes, especially those intended to produce Synthetic Natural Gas feedstocks, eg the British Gas/Lurgi Slagging Gasifier, are designed to produce higher initial $H_2$:CO ratios, eg 3:1, from coal.

The invention will now be described by way of example only.

EXAMPLE 1 -COMPARISON

A variety of catalysts were prepared for initial screening, these being:

(i) 0.25% Rhodium deposited using a low temperature reduction technique on a commercial extruded silica/alumina catalyst support;

(ii) a commercial copper chromite catalyst typically used for reduction of aldehydes to alcohols, containing 33% CuO, 38% $Cr_2O_3$ and 9% BaO;

(iii)a commercial Co Mo hydrodesulphurisation catalyst being 15% $MoO_3$ and 4% Co O and alumina, and presulphided before use (iv) Amminium thiomolybdate, $(NH_4)_2MoO_2S_2$, was prepared by adding ammonium sulphide solution to a solution of ammonium molybdate, cooling and crystallising. 2.9g of crystalline ammonium thiomylbdate was dissolved in 15ml water and added to 10g of a commercial alumina support in two aliquotes with intermediate drying, the product was reduced to $MoS_2$ under hydrogen at 350°C for 4 hours yielding a theoretical catalyst loading of 20% $MoS_2$ on alumina.

(v) An identical procedure was followed to deposit $MoS_2$ on silica.

(vi) An identical solution of ammonium thiomolybdate to that used to prepare catalyst (iv) was added in one quantity to 10g of commercial active carbon and left overnight before reduction as described above. The product contained an estimated 20% $MoS_2$.

The catalysts were tested in this Example and in the following Examples by packing in a stainless steel U-tube reactor immersed in a fluidised sand bath. Technical grade hydrogen and carbon monoxide were used to simulate synthesis gas at various $H_2$:CO ratios. The mixed gases were passed over active carbon and a palladium Deoxo catalyst to remove potentially interfering traces of carbonyls or oxygen. The mixed gases were compressed and metered into the reactor using a mass flow controller. The products from the reactor were passed through a high pressure separator in which liquid products were collected. The residual gases were reduced in pressure and sampled. The liquid gas products were analysed using conventional chromatography methods. The yields of water were calculated by difference, resulting in some negative results. In general, the alcohol mixtures contained less than 10g/1 of water when analysed.

The reaction conditions and products for catalysts i) to v) are shown in Table 1. It can be seen that the rhodium catalyst is essentially inactive, and although copper chromite and CoMo give high conversions, the products are largely valueless $CO_2$. Of the $MoS_2$ catalysts, the carbon supported catalys showed the largest production of alcohols and was selected for further study.

EXAMPLE 2

105 g of ammonium thiomolybdate crystals were dissolved in 350ml of water and added to 600ml of commercial active carbon made from coconut shells and having a surface area of 800m2/g. The mixture was left for 16 hours, during which period the solution was decolourised. Excess liquor was decanted and the product was vacuum dried in a rotary evaporator, at room temperature. A solution of 37g potassium carbonate in 350ml water was used to impregnate the product, which was then again vacuum dried. The resulting catalyst was activated by heating under a stream of synthesis gas for several hours at 425°C. The catalyst was analysed to contain 19.0% $MoS_2$ and 10.3% $K_2CO_3$.

In all the following Examples, the catalyst was obtained in analogous manner, but varying the quantities of starting materials.

EXAMPLE 3

The variation in alcohol production with varying potassium carbonate loading and a fixed $MoS_2$ loading of 17%, was studied. Analysis of the active carbon without any added $K_2CO_3$ showed the presence of 0.5% potassium, calculated as carbonate. The experimental results are shown in Table 2.

EXAMPLE 4

A series of catalysts with varying amounts of $MoS_2$ and $K_2CO_3$ were prepared and tested, and the experimental results are shown in Table 3.

EXAMPLE 5

The effect of reaction temperature on yields and selectivities was studied, using the same catalyst composition, and the results are shown in Table 4.

. EXAMPLE 6

Utilising the catalyst prepared in Example 2, the effect of varying the $H_2$: CO ratio on the product alcohol composition was studied, and the results are shown in Table 5. It is clear that the lower $H_2$:CO ratio favour the formation of higher alcohols over methanol.

4

EXAMPLE 7

A catalyst having a loading of 17.5% $MoS_2$ and 6.4% $K_2CO_3$ was prepared and the variation in selectivity and overall yields of alcohols were studied by varying the reaction temperature from 275 to 300°C and also the GHSV from appoximately 3000 to approximately 5000. The detailed results are shown in Table 6, from which it can be seen that although the selectivities for alcohols are better at 275°C than at 300°C, the yields of alcohols are improved at the higher temperature. At a given temperature, yields improve with higher GHSV but selectivities increase to methanol whereas those to higher alcohols decrease.

EXAMPLE 8

Utilising the catalyst prepared according to Example 2, a long term run was carried out over 100 hours. Preliminary studies had shown that some initial deactivation of the catalyst would be compensated for by an increase in reaction temperature, and a 5°C step-wise increase from an initial reaction temperature of 275°C to 290°C over 70 hours was adopted. After 70 hours, no further increase in temperature was made, although a slight fall-off in catalyst performance was observed. The experimental results are shown in Table 7.

It is believed that the above Examples confirm that the catalyst of the invention demonstrates improved yields over prior suggested catalysts, when utilising the novel combination of essential features, namely:

i) active carbon as the choice of support
ii) method of absorption of $MoS_3$
iii) optimised $MoS_2$ contents
iv) optimised potassium content
v) pretreatment (activation) at 400 to 500°C

TABLE 1

| Catalyst | Rh on SiO2/Al2O3 | CuCrO2 | CoMoS /Al2O3 | MoS2 on Al2O3 | MoS2 on SiO2 | MoS2 on carbon |
|---|---|---|---|---|---|---|
| **OPERATING CONDITIONS** | | | | | | |
| Temp. (°C) | 275 | 300 | 275 | 275 | 275 | 275 |
| Pressure (bar) | 100 | 100 | 100 | 100 | 100 | 100 |
| GHSV (nl/l cat./h | 1266 | 1023 | 1649 | 1352 | 1367 | 1291 |
| H2:CO | 2 | 2 | 2 | 2 | 2 | 2 |
| **CONVERSIONS (/l cat./h)** | | | | | | |
| CO (g) | 1.9 | 85.8 | 126.0 | 82.1 | 26.4 | 43.4 |
| CO (mols) | 0.1 | 3.1 | 4.5 | 2.9 | 0.9 | 1.5 |
| CO (wt% of CO fed) | 0.2 | 13.4 | 12.2 | 11.2 | 3.1 | 5.4 |
| H2 (g) | – | 7.1 | 13.1 | 3.9 | 7.1 | 11.1 |
| H2 (mols/mol CO) | – | 1.2 | 1.5 | 0.7 | 3.8 | 3.6 |
| **YIELDS (g/l cat./h)** | | | | | | |
| C1 | 1.8 | 9.3 | 8.5 | 28.0 | 3.7 | 3.3 |
| C2 | – | 1.2 | 21.9 | 9.0 | 1.7 | 1.5 |
| C3 | – | 0.0 | 8.7 | 2.7 | 0.0 | 2.3 |
| C4+ | – | 0.0 | 3.8 | 0.0 | 0.0 | 0.0 |
| CO2 | – | 82.3 | 96.2 | 42.4 | 15.1 | 20.3 |
| MeOH | – | 0.0 | 0.0 | 2.2 | 11.1 | 21.9 |
| EtOH | – | 0.0 | 0.0 | 1.2 | 1.6 | 4.1 |
| PrOH | – | 0.0 | 0.0 | 0.5 | 0.4 | 0.5 |
| H2O | – | 0.0 | 0.0 | 0.0 | 0.0 | 0.7 |
| Total | 1.8 | 92.9 | 139.1 | 86.0 | 33.5 | 54.5 |
| **SELECTIVITIES** | | | | | | |
| C1 | 96.2 | 10.9 | 6.7 | 34.1 | 13.8 | 7.6 |
| C2 | – | 1.5 | 17.3 | 11.0 | 6.5 | 3.5 |
| C3 | – | 0.0 | 6.9 | 3.2 | 0.0 | 5.2 |
| C4+ | – | 0.0 | 3.0 | 0.0 | 0.0 | 0.0 |
| CO2 | – | 96.0 | 76.3 | 51.7 | 57.1 | 46.8 |
| MeOH | – | 0.0 | 0.0 | 2.6 | 41.9 | 50.4 |
| EtOH | – | 0.0 | 0.0 | 1.5 | 6.2 | 9.4 |
| PrOH | – | 0.0 | 0.0 | 0.6 | 1.3 | 1.1 |
| H2O | – | 0.0 | 0.0 | 0.0 | 0.0 | 1.6 |
| H2 | – | –8.3 | 10.41 | –4.7 | –26.9 | –25.7 |
| Total | – | 100.0 | 100.0 | 100.0 | 100.0 | 100.0 |
| **SELECTIVITIES (CO2 free, wt.% of CO)** | | | | | | |
| C1–C4 | 100.0 | 100.0 | 100.0 | 91.0 | 29.1 | 20.7 |
| MeOH | – | 0.0 | 0.0 | 5.0 | 60.0 | 63.9 |
| EtOH | – | 0.0 | 0.0 | 2.8 | 8.9 | 11.9 |
| PrOH | – | 0.0 | 0.0 | 1.2 | 1.9 | 1.4 |
| Total | 100.0 | 100.0 | 100.0 | 100.0 | 100.0 | 100.0 |

## TABLE 2

| K2CO3 LOADING (%w/w) | 0.50 | 3.40 | 6.40 | 9.40 | 10.30 | 15.00 |
|---|---|---|---|---|---|---|
| **OPERATING CONDITIONS** | | | | | | |
| Temp. (°C) | 275 | 275 | 275 | 275 | 275 | 275 |
| Pressure (bar) | 100 | 100 | 100 | 100 | 100 | 100 |
| GHSV (nl/l cat./h | 3436 | 3391 | 3279 | 3444 | 3436 | 3540 |
| H2:CO | 2 | 2 | 2 | 2 | 2 | 2 |
| Recoveries (%) | 106.2 | 104.7 | 102.2 | 104.7 | 106.1 | 109.7 |
| **CONVERSIONS (/l cat./h)** | | | | | | |
| CO (g) | 112.6 | 360.7 | 448.0 | 560.9 | 314.9 | 256.2 |
| CO (mols) | 4.0 | 12.9 | 16.0 | 20.0 | 11.2 | 9.2 |
| CO (wt% of CO fed) | 4.1 | 13.8 | 18.4 | 22.7 | 13.0 | 9.8 |
| H2 (g) | 46.1 | 50.6 | 57.8 | 73.8 | 47.7 | 47.8 |
| H2 (mols/mol CO) | 5.7 | 2.0 | 1.8 | 1.8 | 2.1 | 2.6 |
| **YIELDS (g/l cat./h)** | | | | | | |
| C1 | 9.1 | 24.1 | 28.5 | 37.9 | 18.6 | 15.1 |
| C2 | 0.0 | 0.0 | 0.0 | 0.0 | 7.0 | 0.0 |
| C3 | 0.0 | 0.0 | 0.0 | 0.0 | 0.0 | 0.0 |
| C4+ | 0.0 | 0.0 | 0.0 | 0.0 | 0.0 | 0.0 |
| CO2 | 62.4 | 132.8 | 147.0 | 198.9 | 102.0 | 103.9 |
| MeOH | 66.1 | 179.6 | 246.2 | 317.1 | 192.4 | 161.2 |
| EtOH | 17.9 | 65.8 | 62.9 | 65.6 | 46.9 | 29.7 |
| PrOH | 4.1 | 11.4 | 12.1 | 12.6 | 8.3 | 6.9 |
| H20 | -0.9 | -2.5 | 9.1 | 2.6 | -12.6 | -12.7 |
| Total | 158.7 | 411.2 | 505.8 | 634.6 | 362.6 | 304.0 |
| **SELECTIVITIES** | | | | | | |
| C1 | 8.1 | 6.7 | 6.4 | 6.8 | 5.9 | 5.9 |
| C2 | 0.0 | 0.0 | 0.0 | 0.0 | 2.2 | 0.0 |
| C3 | 0.0 | 0.0 | 0.0 | 0.0 | 0.0 | 0.0 |
| C4+ | 0.0 | 0.0 | 0.0 | 0.0 | 0.0 | 0.0 |
| CO2 | 55.5 | 36.8 | 32.8 | 35.5 | 32.4 | 40.5 |
| MeOH | 58.7 | 49.8 | 55.0 | 56.5 | 61.1 | 62.9 |
| EtOH | 15.9 | 18.2 | 14.1 | 11.7 | 14.9 | 11.6 |
| PrOH | 3.6 | 3.2 | 2.7 | 2.2 | 2.6 | 2.7 |
| H20 | -0.8 | -0.7 | 2.0 | 0.5 | -4.0 | -5.0 |
| H2 | -41.0 | -14.0 | -12.9 | -13.2 | -15.1 | -18.6 |
| Total | 100.0 | 100.0 | 100.0 | 100.0 | 100.0 | 100.0 |
| **SELECTIVITIES** (CO2 free, wt.% of CO) | | | | | | |
| C1-C4 | 9.4 | 8.7 | 7.9 | 8.7 | 9.8 | 7.6 |
| MeOH | 68.7 | 64.5 | 68.6 | 72.8 | 73.8 | 80.5 |
| EtOH | 18.6 | 23.6 | 17.5 | 15.1 | 18.0 | 14.8 |
| PrOH | 4.2 | 4.1 | 3.4 | 2.9 | 3.2 | 3.4 |
| H20 | -1.0 | -0.9 | 2.5 | 0.6 | -4.8 | -6.3 |
| Total | 100.0 | 100.0 | 100.0 | 100.0 | 100.0 | 100.0 |

## TABLE 3

| CATALYST COMPOSITION | | | | | |
|---|---|---|---|---|---|
| MoS2 | 1.03 | 5.2 | 14.5 | 18.2 | 22.2 |
| K2CO3 | 1.9 | 3.9 | 5.5 | 9.4 | 12 |
| CATALYST VOL. (cc) | 15 | 15 | 15 | 15 | 15 |
| | | | | | |
| OPERATING CONDITIONS | | | | | |
| Temp. (°c) | 275 | 275 | 275 | 275 | 275 |
| Pressure (bar) | 100 | 100 | 100 | 100 | 100 |
| GHSV (nl/l cat./h | 3334 | 3487 | 3389 | 3451 | 3362 |
| H2:CO | 2 | 2 | 2 | 2 | 2 |
| Recoveries (%) | 102.6 | 107.7 | 109.8 | 104.9 | 104.1 |
| | | | | | |
| CONVERSIONS (/l cat./h) | | | | | |
| CO (g) | -11.6 | 178.9 | 283.0 | 547.6 | 488.7 |
| CO (mols) | - 0.4 | 6.4 | 10.0 | 19.6 | 17.5 |
| CO (wt% of CO fed) | - 0.3 | 5.3 | 10.4 | 22.9 | 23.1 |
| H2 (g) | -11.6 | 30.9 | 65.0 | 72.1 | 58.4 |
| H2 (mols/mol CO) | -14.0 | 2.4 | 3.2 | 1.8 | 1.7 |
| | | | | | |
| YIELDS (g/l cat./h) | | | | | |
| C1 | 0.0 | 10.9 | 4.0 | 36.6 | 35.7 |
| C2 | 0.0 | 0.0 | 0.0 | 6.2 | 5.6 |
| C3 | 0.0 | 0.0 | 0.0 | 0.0 | 0.0 |
| C4+ | 0.0 | 0.0 | 0.0 | 0.0 | 0.0 |
| CO2 | 0.0 | 60.2 | 99.9 | 192.3 | 180.2 |
| | | | | | |
| MeOH | 0.0 | 104.7 | 197.6 | 306.5 | 245.6 |
| EtOH | 0.0 | 28.9 | 40.6 | 63.5 | 53.4 |
| PrOH | 0.0 | 5.3 | 7.9 | 12.1 | 11.7 |
| H2O | 0.0 | - 0.1 | - 2.1 | 2.5 | 14.9 |
| Total | 0.0 | 209.8 | 348.0 | 619.7 | 547.1 |
| | | | | | |
| SELECTIVITIES | | | | | |
| C1 (wt.% of CO) | 0.0 | 6.1 | 1.4 | 6.7 | 7.3 |
| C2 | 0.0 | 0.0 | 0.0 | 1.1 | 1.1 |
| C3 | 0.0 | 0.0 | 0.0 | 0.0 | 0.0 |
| C4+ | 0.0 | 0.0 | 0.0 | 0.0 | 0.0 |
| CO2 | 0.0 | 33.6 | 35.3 | 35.0 | 36.9 |
| MeOH | 0.0 | 58.5 | 69.8 | 56.0 | 50.2 |
| EtOH | 0.0 | 16.1 | 14.4 | 11.6 | 10.9 |
| PrOH | 0.0 | 2.9 | 2.8 | 2.2 | 2.4 |
| H2O | 0.0 | -0.1 | -0.8 | 0.5 | 3.0 |
| H2 | 100.0 | -17.3 | -23.0 | -13.2 | -11.9 |
| Total | 100.0 | 100.0 | 100.0 | 100.0 | 100.0 |
| | | | | | |
| SELECTIVITIES (CO2 free,wt.% of CO) | | | | | |
| C1-C4 | 0.0 | 7.3 | 1.6 | 10.0 | 11.3 |
| MeOH | 0.0 | 70.0 | 79.7 | 71.7 | 66.9 |
| EtOH | 0.0 | 19.3 | 16.4 | 14.8 | 14.6 |
| PrOH | 0.0 | 3.5 | 3.2 | 2.8 | 3.2 |
| H2O | 0.0 | -0.1 | -0.9 | 0.6 | 4.1 |
| Total | 0.0 | 100.0 | 100.0 | 100.0 | 100.0 |

8

TABLE 4

| CATALYST | 17% | MoS2 on Carbon (10% K2CO3) | | | | |
|---|---|---|---|---|---|---|
| CATALYST VOL. (cc) | 15 | 15 | 15 | 15 | 15 | 15 |
| **OPERATING CONDITIONS** | | | | | | |
| Temp. (°c) | 275 | 290 | 300 | 310 | 325 | 350 |
| Pressure (bar) | 100 | 100 | 100 | 100 | 100 | 100 |
| GHSV (nl/l cat./h | 5328 | 5189 | 5303 | 5293 | 5304 | 5283 |
| H2:CO | 2 | 2 | 2 | 2 | 2 | 2 |
| Recoveries (%) | 106.0 | 103.6 | 105.1 | 105.7 | 105.9 | 102.0 |
| **CONVERSIONS (/l cat./h)** | | | | | | |
| CO (g) | 215.3 | 410.3 | 564.9 | 685.5 | 931.0 | 1316.7 |
| CO (mols) | 7.7 | 14.7 | 20.2 | 24.5 | 33.3 | 47.0 |
| CO (wt% of CO fed) | 5.3 | 10.4 | 14.0 | 17.0 | 23.1 | 32.8 |
| H2 (g) | 20.3 | 24.5 | 38.6 | 67.7 | 82.7 | 102.1 |
| H2 (mols/mol CO) | 1.3 | 0.8 | 1.0 | 1.4 | 1.2 | 1.1 |
| **YIELDS (g/l cat./h)** | | | | | | |
| C1 | 6.3 | 18.4 | 24.3 | 40.0 | 66.5 | 144.3 |
| C2 | 0.0 | 0.0 | 11.4 | 10.7 | 10.4 | 30.1 |
| C3 | 0.0 | 0.0 | 0.0 | 0.0 | 0.0 | 14.7 |
| C4+ | 0.0 | 0.0 | 0.0 | 0.0 | 0.0 | 0.0 |
| CO2 | 69.5 | 134.8 | 183.9 | 235.8 | 350.6 | 646.5 |
| MeOH | 120.1 | 213.4 | 294.7 | 374.9 | 471.6 | 472.1 |
| EtOH | 27.5 | 49.9 | 67.0 | 73.5 | 84.6 | 70.3 |
| PrOH | 5.5 | 11.4 | 14.0 | 21.0 | 30.2 | 44.7 |
| H20 | 6.6 | 6.8 | 8.1 | -2.7 | -0.2 | - 3.8 |
| Total | 235.6 | 434.8 | 603.6 | 753.2 | 1013.8 | 1418.7 |
| **SELECTIVITIES** | | | | | | |
| C1 (wt.% of CO) | 2.9 | 4.5 | 4.3 | 5.8 | 7.1 | 11.0 |
| C2 | 0.0 | 0.0 | 2.0 | 1.6 | 1.1 | 2.3 |
| C3 | 0.0 | 0.0 | 0.0 | 0.0 | 0.0 | 1.1 |
| C4+ | 0.0 | 0.0 | 0.0 | 0.0 | 0.0 | 0.0 |
| CO2 | 32.3 | 32.8 | 32.6 | 34.4 | 37.7 | 49.1 |
| MeOH | 55.8 | 52.0 | 52.2 | 54.7 | 50.7 | 35.9 |
| EtOH | 12.8 | 12.2 | 11.9 | 10.7 | 9.1 | 5.3 |
| PrOH | 2.6 | 2.8 | 2.5 | 3.1 | 3.2 | 3.4 |
| H20 | 3.1 | 1.7 | 1.4 | -0.4 | 0.0 | -0.3 |
| H2 | -9.4 | - 6.0 | - 6.8 | - 9.9 | - 8.9 | -7.8 |
| Total | 100.0 | 100.0 | 100.0 | 100.0 | 100.0 | 100.0 |
| **SELECTIVITIES** | | | | | | |
| **(CO2 free,wt.% of CO)** | | | | | | |
| C1-C4 | 3.8 | 6.1 | 8.5 | 9.8 | 11.6 | 24.5 |
| MeOH | 72.3 | 71.2 | 70.2 | 72.5 | 71.1 | 61.1 |
| EtOH | 16.6 | 16.6 | 16.0 | 14.2 | 12.8 | 9.1 |
| PrOH | 3.3 | 3.8 | 3.3 | 4.1 | 4.6 | 5.8 |
| H20 | 4.0 | 2.3 | 1.9 | -0.5 | 0.0 | - 0.5 |
| Total | 100.0 | 100.0 | 100.0 | 100.0 | 100.0 | 100.0 |

TABLE 5

Composition (wt %)

| | 0.3 | 1 | 2 | 3 |
|---|---|---|---|---|
| $H_2$: CO | 0.3 | 1 | 2 | 3 |
| Temp (°C) | 275 | 275 | 275 | 275 |
| Methanol | 46.0 | 63.7 | 80.1 | 84.4 |
| Ethanol | 38.9 | 28.8 | 16.7 | 13.4 |
| Propanol | 15.3 | 7.4 | 3.2 | 2.2 |
| Temp °C | 300 | 300 | 300 | 300 |
| Methanol | 30.0 | 57.0 | 71.7 | 80.7 |
| Ethanol | 44.3 | 33.4 | 21.5 | 15.5 |
| Propanol | 25.7 | 9.6 | 6.8 | 3.8 |

## TABLE 6

| SAMPLE NUMBER | 1 | 2 | 3 | 4 |
|---|---|---|---|---|
| HOURS ON STREAM | 2 | 4 | 6 | 8 |
| CATALYST VOL. (cc) | 15 | 15 | 15 | 15 |
| | | | | |
| OPERATING CONDITIONS | | | | |
| Temp. (°C) | 300 | 300 | 275 | 275 |
| Pressure (bar) | 100 | 100 | 100 | 100 |
| GHSV (nl/l cat./h | 3231 | 5257 | 3286 | 5243 |
| H2:CO | 2 | 2 | 2 | 2 |
| Recoveries (%) | 102.3 | 106.2 | 102.2 | 106.0 |
| | | | | |
| CONVERSIONS (/Kg cat./h) | | | | |
| CO (g) | 1096.6 | 1309.8 | 448.4 | 467.4 |
| CO (mols) | 39.2 | 46.8 | 16.0 | 16.7 |
| CO (wt% of CO fed) | 45.7 | 33.5 | 18.4 | 12.0 |
| H2 (g) | 109.8 | 144.5 | 57.9 | 60.0 |
| H2 (mols/mol CO) | 1.4 | 1.5 | 1.8 | 1.8 |
| | | | | |
| YIELDS (g/Kg cat./h) | | | | |
| C1 | 113.1 | 115.8 | 28.6 | 24.3 |
| C2 | 22.9 | 19.7 | 0.0 | 0.0 |
| C3 | 8.4 | 14.5 | 0.0 | 0.0 |
| C4+ | 0.0 | 0.0 | 0.0 | 0.0 |
| CO2 | 563.2 | 593.5 | 147.4 | 150.6 |
| MeOH | 242.1 | 452.3 | 246.2 | 278.0 |
| EtOH | 152.3 | 175.7 | 62.9 | 64.0 |
| PrOH | 57.6 | 54.2 | 12.1 | 12.0 |
| H2O | 46.7 | 28.6 | 9.1 | −1.6 |
| Total | 1206.3 | 1454.3 | 506.3 | 527.4 |
| | | | | |
| SELECTIVITIES | | | | |
| C1 (wt.% of CO) | 10.3 | 8.8 | 6.4 | 5.2 |
| C2 | 2.1 | 1.5 | 0.0 | 0.0 |
| C3 | 0.8 | 1.1 | 0.0 | 0.0 |
| C4+ | 0.0 | 0.0 | 0.0 | 0.0 |
| CO2 | 51.4 | 45.3 | 32.9 | 32.2 |
| MeOH | 22.1 | 34.5 | 54.9 | 59.5 |
| EtOH | 13.9 | 13.4 | 14.0 | 13.7 |
| PrOH | 5.3 | 4.1 | 2.7 | 2.6 |
| H2O | 4.3 | 2.2 | 2.0 | −0.3 |
| H2 | −10.0 | −11.0 | −12.9 | −12.8 |
| Total | 100.0 | 100.0 | 100.0 | 100.0 |
| | | | | |
| SELECTIVITIES | | | | |
| (CO2 free,wt.% of CO) | | | | |
| C1-C4 | 22.5 | 17.4 | 8.0 | 6.5 |
| MeOH | 37.6 | 52.5 | 68.6 | 73.8 |
| EtOH | 23.7 | 20.4 | 17.5 | 17.0 |
| PrOH | 9.0 | 6.3 | 3.4 | 3.2 |
| H2O | 7.3 | 3.3 | 2.5 | −0.4 |
| Total | 100.0 | 100.0 | 100.0 | 100.0 |

## TABLE 7

| HOURS ON STREAM | 9 | 24 | 49 | 71 | 100 |
|---|---|---|---|---|---|
| CATALYST VOL. (cc) | 15 | 15 | 15 | 15 | 15 |
| **OPERATING CONDITIONS** | | | | | |
| Temp. (°C) | 275 | 280 | 285 | 290 | 290 |
| Pressure (bar) | 100 | 100 | 100 | 100 | 100 |
| GHSV (nl/l cat./h | 3272 | 3363 | 3354 | 3152 | 3446 |
| H2:CO | 2 | 2 | 2 | 2 | 2 |
| Recoveries (%) | 102.2 | 101.4 | 101.9 | 96.1 | 105.3 |
| **CONVERSIONS (/Kg cat./h)** | | | | | |
| CO (g) | 352.7 | 342.5 | 337.4 | 405.7 | 322.5 |
| CO (mols) | 12.6 | 12.2 | 12.0 | 14.5 | 11.5 |
| CO (wt% of CO fed) | 15.0 | 13.9 | 13.7 | 17.6 | 12.3 |
| H2 (g) | 35.8 | 29.7 | 43.8 | 43.2 | 34.4 |
| H2 (mols/mol CO) | 1.4 | 1.2 | 1.8 | 1.5 | 1.5 |
| **YIELDS (g/Kg cat./h)** | | | | | |
| C1 | 14.4 | 18.9 | 18.2 | 30.6 | 15.3 |
| C2 | 6.8 | 0.0 | 0.0 | 0.0 | 0.0 |
| C3 | 0.0 | 0.0 | 0.0 | 0.0 | 0.0 |
| C4+ | 0.0 | 0.0 | 0.0 | 0.0 | 0.0 |
| CO2 | 119.1 | 114.2 | 120.4 | 168.3 | 115.9 |
| MeOH | 193.2 | 197.5 | 192.7 | 215.3 | 181.8 |
| EtOH | 41.8 | 44.3 | 43.6 | 47.4 | 41.0 |
| PrOH | 7.3 | 9.3 | 9.5 | 7.9 | 9.5 |
| H2O | 5.8 | -12.0 | -3.2 | -20.6 | -6.5 |
| Total | 388.5 | 372.2 | 381.2 | 448.9 | 356.9 |
| H2O g/l a.d. | 11.9 | 11.7 | 10.8 | 10.6 | 10.4 |
| **SELECTIVITIES** | | | | | |
| C1 (wt.% of CO) | 4.1 | 5.5 | 5.4 | 7.5 | 4.8 |
| C2 | 1.9 | 0.0 | 0.0 | 0.0 | 0.0 |
| C3 | 0.0 | 0.0 | 0.0 | 0.0 | 0.0 |
| C4+ | 0.0 | 0.0 | 0.0 | 0.0 | 0.0 |
| CO2 | 33.8 | 33.4 | 35.7 | 41.5 | 35.9 |
| MeOH | 54.8 | 57.7 | 57.1 | 53.1 | 56.4 |
| EtOH | 11.9 | 12.9 | 12.9 | 11.7 | 12.7 |
| PrOH | 2.1 | 2.7 | 2.8 | 1.9 | 2.9 |
| H2O | 1.7 | -3.5 | -1.0 | -5.1 | -2.0 |
| H2 | -10.2 | -8.7 | -13.0 | -10.6 | -10.7 |
| Total | 100.0 | 100.0 | 100.0 | 100.0 | 100.0 |
| **SELECTIVITIES** | | | | | |
| **(CO2 free,wt.% of CO)** | | | | | |
| C1-C4 | 7.9 | 7.3 | 7.0 | 10.9 | 6.4 |
| MeOH | 71.7 | 76.5 | 73.9 | 76.7 | 75.4 |
| EtOH | 15.5 | 17.2 | 16.7 | 16.9 | 17.0 |
| PrOH | 2.7 | 3.6 | 3.6 | 2.8 | 3.9 |
| H2O | 2.2 | -4.6 | -1.2 | -7.3 | -2.7 |
| Total | 100.0 | 100.0 | 100.0 | 100.0 | 100.0 |

**Claims**

1. A promoted MoS₂ catalyst produced by contacting an active carbon support with an aqueous solution of sufficient concentration of ammonium thiomolybdate to give a loading of 10 to 25% by wt of MoS₂ in the final catalyst, by analysis, at a temperature of 0 to 50°C, depositing sufficient of potassium carbonate or hydroxide, or a potassium salt other than a salt containing chlorine in its anion, to give a loading of 5 to 12% by wt, calculated as K₂CO₃, and activating the catalyst by heating in a reducing atmosphere at a temperature of 400 to 500°C.

2. A catalyst according to claim 1, wherein the loading of MoS₂ is in the range 15 to 20% by wt.

3. A catalyst according to claim 1 or 2, wherein the loading of potassium compound is in the range 6 to 10% by wt, calculated as K₂CO₃.

4. A catalyst according to any one of claim 1 to 3, wherein the carbon support has a surface area of at least 500m²/g.

5. A catalyst according to any one of the preceding claims, wherein the activation temperature is in the range 425 to 450°C.

6. A catalyst according to claim 1, substantially as hereinbefore described.

7. A process for the production of a promoted MoS₂ catalyst, comprising contacting an active carbon support with an aqueous solution of sufficient concentration of ammonium thiomolybdate to give a loading of 10 to 25% by wt of MoS₂ in the product catalyst, by analysis, at a temperature of 0 to 50°C, depositing sufficient of potassium carbonate or hydroxide, or a potassium salt other than a salt containing chlorine in its anion, to give a loading of 5 to 12% by wt, calculated as K₂CO₃, and activating the catalyst by heating in a reducing atmosphere at a temperature of 400 to 500°C.

8. A process according to claim 7, wherein the activation is carried out at a temperature from 425 to 450°C.

9. A process according to claim 7 or 8, wherein the reducing atmosphere in the activation us synthesis gas.

10. A process according to claim 7, substantially as hereinbefore described.

11. A catalyst prepared by a process according to any one of claims 7 to 10.

12. A process for the production of mixed alcohols comprising the passage of a mixture of hydrogen and carbon monoxide in a volume ratio of from 0.2 to 2:1 over a catalyst according to any one of claims 1 to 6 or 11, at a temperature of 260 to 325°C and a pressure of at least 50 bar, and at a Gas Hourly Space Velocity of from 1000 to 50,000 h⁻¹.

13. A process according to claim 12, wherein the volume ratio in the synthesis gas is from 0.5 to 1:1.

14. A process according to claim 12 or 13, wherein the catalyst temperature is from 275 to 300°C.

15. A process according to claim 12, 13 or 14, wherein the GHSV is from 5,000 to 20,000 h⁻¹.

16. A process according to any one of claims 12 to 15, wherein the pressure is 100 to 150 bar.

17. A process according to claim 12, substantially as hereinbefore described.

18. Mixed alcohols whenever produced by a process according to any one of claims 12 to 17.

19. Mixed alcohols according to claim 18, blended with gasoline.